# EUROPEAN PATENT APPLICATION

(11) **EP 1 097 989 A2**
(43) Date of publication of application: **09.05.2001**
(21) Application number: 00118272.4
(22) Date of filing: 05.09.2000
(51) Int. Cl.: C12N 9/12

(54) **Ndr phosphokinase**

(30) Priority: 07.09.1999 GB 9921121
(71) Applicant: Novartis AG, 4058 Basel (CH); Novartis-Erfindungen Verwaltungsgesellschaft m.b.H., 1230 Wien (AT)
(72) Inventor: Millward, Thomas Anders, 4054 Basel (CH); Hemmings, Brian Arthur, 4126 Bettingen (CH)
(74) Representative: Becker, Konrad

(57) **Abstract**

The present invention relates to the phosphorylated form of nuclear serine/threonine protein kinase, designated nuclear, Dbf2-related kinase (Ndr), and provides assays and materials for identifying modulators thereof. The invention relates to the fields of molecular biology, chemistry, pharmacology, and screening technology. Normal text

## Description

The present invention relates to the phosphorylated form of nuclear serine/threonine protein kinase, designated nuclear, Dbf2-related kinase (Ndr), and provides assays and materials for identifying modulators thereof. The invention relates to the fields of molecular biology, chemistry, pharmacology, and screening technology.

Reversible protein phosphorylation is a major mechanism for the co-ordinated control of many fundamental cellular functions in eukaryotic organisms, including metabolism, growth, and differentiation. The phosphorylation status, and consequently the activity, of specific target proteins is regulated by the opposing actions of protein kinases and protein phosphatases. Generally, these enzymes are specific either for serine/threonine or for tyrosine phosphoacceptors, although some dual specificity kinases and phosphatases have also been described. The importance of phosphorylation cascades is reflected by the finding that many kinases, phosphatases, and the signal transduction pathways in which they participate have been highly conserved during the course of evolution. In recent years, interest has focused on the role of protein phosphorylation in the control of the cell cycle; a number of cellular proto-oncogenes encode members of the serine/threonine kinase family and it has become increasingly clear that certain serine/threonine kinases function as key components of the cell cycle regulatory network. Therefore, the complete delineation of these pathways is an important aim for the understanding of oncogenesis and tumour progression.

Ndr-protein kinase (also known as Pun kinase) is a nuclear serine/threonine protein kinase that is expressed in almost all cell types of the body (Millward, T. et al. (1995) *Proc. Natl*. *Acad. Sci. USA* 92, 5022-5026). WO 96/19579, herein incorporated by reference, describes the identification of Ndr kinase, an *in vitro* autophosphorylated form of Ndr kinase, antibodies specific for Ndr kinase, useful methods for making and using Ndr kinase, as well as other related aspects of Ndr kinase.

Ndr belongs to a subfamily (or subgroup) of kinases that includes the kinases LATS, Orb6, Cot-1 and Dbf2. Sequence comparisons show that Ndr is most closely related to other members of said subfamily of kinases, such as LATS, Orb6 and Cot-1 (50 - 60% catalytic domain identity with Ndr) and Dbf2 (40% catalytic domain identity with Ndr). The Ndr subfamily of kinases has been implicated in the regulation of cell growth, cell division and cell morphology.

For example, LATS (also called Wts) was originally discovered as a gene that, when deleted, caused an overgrowth phenotype in Drosophila, and was thus proposed to be a tumor suppressor gene (Justice, R. W. et al. (1995) *Genes Dev*. **9**, 534-546; Xu, T. et al. (1995) *Development* **121**, 1053-1063). Recently, the mammalian LATS homologue was cloned, and its tumor suppressor function was confirmed. Homozygous deletion of the LATS homologue in mice causes the development of soft tissue sarcomas and ovarian stromal cell tumors (St. John, M. A. et al. (1999) *Nat. Genet*. **21**, 182-186).

Other members of the Ndr kinase family have been demonstrated to be of importance in cell division. In fission yeast, Orb6 is required for the establishment of cell polarity and also appears to act as an inhibitor of Cdc2 (Verde, F. et al. (1998) *Proc. Natl. Acad. Sci. USA* 95, 7526-7531). Like Orb6, Cot-1 from Neurospora crassa is also required for polarized cell growth (Yarden, O. et al. (1992) *EMBO J*. 11, 2159-2166). Finally, Dbf2 is part of a pathway required for downregulation of Cdc28-cyclin B kinase activity at the end of mitosis in budding yeast (Jaspersen, S. L. et al. (1998) *Mol. Biol. Cell.* 9, 2803-2817).

The Ndr kinase subfamily is clearly important in cell growth control but it is not known how each kinase is regulated. Some members of the Ndr kinase family are known to exist as phosphoproteins (Toyn, J. H. and Johnston, L. H. (1994) *EMBO J*. 13, 1103-1113; Tao et al. (1999) *Nature Genetics* 21, 177-181) but phosphorylation has not been directly demonstrated to play a role in their regulation.

### Summary of the invention

The present invention relates to nuclear, Dbf2-related (Ndr) phosphokinase, a functional homologue thereof or a modulator thereof, wherein said Ndr phosphokinase is phosphorylated at Ser-281 and/or Thr-444.

Furthermore, the present invention relates to an antibody that specifically binds to Ndr phosphokinase or a functional homologue thereof.

The present invention also relates to a method for producing the Ndr phosphokinase, said method comprising treating cells containing a nucleic acid encoding Ndr protein kinase with okadaic acid, and allowing said cells to express said Ndr protein kinase.

In another aspect the invention relates to a method for screening potential modulators of Ndr kinase activity, said method comprising the steps of a) incubating Ndr phosphokinase or a functional homologue thereof with the potential modulator and b) detecting interaction between the modulator and Ndr phosphokinase or the functional homologue thereof.

The present invention also relates to a method for screening potential modulators of Ndr kinase activity, said method comprising the steps of a) incubating Ndr kinase or a functional homologue thereof with the potential modulator and b) detecting a change in phosphorylation of said Ndr kinase or the functional homologue thereof.

In a yet further aspect of the present invention a method of identifying an Ndr kinase substrate is provided, said method comprising the steps of (a) incubating Ndr phosphokinase or a functional homologue thereof with the potential substrate and (b) detecting a change in phosphorylation of said Ndr kinase substrate, with the proviso that said Ndr kinase substrate is not Ndr kinase.

In another aspect the invention relates to the use of Ndr phosphokinase, or a functional homologue thereof, for the identification of an agonist or antagonist of Ndr kinase activity.

In a further aspect the invention relates to the use of Ndr phosphokinase, a functional homologue thereof or a modulator therof, as a medicament.

In a yet further aspect the invention relates to the use of Ndr phosphokinase, a functional homologue thereof or a modulator therof, in the treatment of abnormalities in cellular proliferation.

Furthermore, the present invention relates to a kit comprising Ndr phosphokinase or a functional fragment thereof and instructions.

In another aspect the present invention relates to the present invention relates to a phosphokinase of the subfamily of Ndr-related phosphokinases, a functional homologue thereof or a modulator thereof, wherein said phosphokinase is phosphorylated at amino acids Ser-281 and/or Thr-444 of Ndr protein kinase or amino acids corresponding to amino acids Ser-281 and/or Thr-444 of Ndr protein kinase.

### Detailed description of the invention

The present invention is directed to the Ndr phosphokinase or phosphorylated fragments and functional homologues thereof, antibodies that bind specifically thereto, their uses and to methods of producing phosphorylated Ndr kinase. As used herein, the terms "phosphorylated Ndr protein kinase", "phosphorylated Ndr kinase" and "Ndr phosphokinase" are used interchangeably.

Homologues are polypeptides which share sufficient similarities for the skilled person to determine that they share homology of structure or function. This includes all species homologues from other organisms that may be isolated according to conventional methods. A "functional homologue" or a "functional equivalent" of a polypeptide (or peptide), includes molecules derived from the native polypeptide sequence, for example, provided in WO 96/19579, as well as polypeptides which function in a manner similar to the reference molecule to achieve a desired result. Thus, a functional homologue of Ndr phosphokinase encompasses derivatives and analogues of those polypeptides, including any single or multiple amino acid additions, substitutions and/or deletions occurring internally (with the exception of both Ser-281 and Thr-444) or at the amino or carboxy termini thereof - so long as Ndr phosphokinase activity remains. Similarly, a functional homologue includes functional fragments of the polypeptide, as long as the activity of the polypeptide remains. Smaller peptides containing the biological activity of polypeptide are included in the invention.

As used herein, the term "Ndr kinase activity" is not limited to the ability of Ndr phosphokinase to phosphorylate a substrate but also includes other characteristics of the protein or peptide, such as, their ability to bind an antibody specific for Ndr phosphokinase (i.e., differentiate between the phosphorylated and non-phosphorylated state of Ndr kinase), or their ability to bind to a substrate or a modulator of Ndr kinase.

Potential phosphorylation sites on Ndr kinase are numerous. Phosphoserine and phosphothreonine residues have been identified in a phosphoamino acid analysis of in vitro autophosphorylated, recombinant glutathione-S-transferase - Ndr kinase fusion protein WO 96/19579. However, autophosphorylation is achieved under artificial in vitro assay conditions, where the Ndr kinase substrate is absent and ATP concentrations are high, and may not reflect the in vivo situation. Thus, in one aspect of the present invention, the phosphorylation sites of the in vivo phosphorylated Ndr kinase are provided.

A number of kinases are known to exist in phosphorylated forms in vivo. Typically, to isolate a phosphokinase, cells expressing kinase are treated with any of various agents known to result in higher levels of phosphokinase. These agents include serum, mitogens and/or activating agents, such as IL-2, platelet-derived growth factor (PDGF), insulin, epidermal growth factor (EGF), basic fibroblast growth factor (bFGF), and phosphatase inhibitors, such as vanadate and okadaic acid (WO97/22716). However, the preparation of sufficient quantities of Ndr phosphokinase to map the phosphorylation sites was not routine.

Initially, an attempt was made to map the phosphorylation sites of the autophosphorylated Ndr kinase but autophosphorylated Ndr phosphokinase levels were too low (less than 1% phosphorylation was achieved). Various techniques, all of which are commonly used to activate other kinases, were used to increase the abundance of Ndr phosphokinase in vivo, without success. In particular, the following cell treatments failed to activate Ndr kinase: mitogenic stimuli (including serum, EGF, insulin, insulin-like growth factor (IGF-1), bradykinin, bombesin and vasopressin), stress-inducing agents (including hyperosmotic shock using NaCl or sorbitol, anisomysin and hydrogen peroxide), cyclosporin A, and the phosphatase inhibitor, vanadate. Finally, okadaic acid (phosphatase inhibitor) was used in culture and sufficient phosphokinase was obtained to map the in vivo phosphorylation sites of Ndr kinase (See Example 1, below).

Native Ndr kinase may can be induced in this manner. Alternatively, an expression vector comprising the Ndr kinase coding sequence can be introduced into cells which then express Ndr kinase.

The DNA coding for Ndr kinase may be comprised in a nucleic acid expression cassette comprising a promoter operably linked to a nucleic acid as defined above and optionally to transcription termination signals.

The promoter can be of almost any origin. It is for example possible to use a tightly regulated promoter or the promoter that is naturally adjacent to the Ndr gene. Preferred are promoters that are active in the chosen host cells like the SV40, tac, *β*-actin, metallothionein, T7, polyhedrin and cytomegalovirus promoter.

A DNA sequence containing the transcription termination signals is preferably the 3' flanking sequence of a gene that contains proper signals for transcription termination and polyadenylation for the desired host. Suitable signals are, for example, the polyadenylation signal of the human growth hormone, of the DHFR gene and of the rabbit *β*-globin gene.

It is also possible to use a polypeptide expression cassette additionally containing a signal sequence, that causes the protein produced to be secreted into the medium. Suitable signal sequences are known in the art. Accordingly, in these kinds of expression cassettes a promoter is operably linked to a first DNA sequence encoding a signal peptide linked in the proper reading frame to a second DNA sequence coding for Ndr kinase, and a DNA sequence containing transcription termination signals.

The promoter, the DNA sequence coding for Ndr kinase and the DNA sequence containing transcription termination signals are operably linked to each other, i.e., they are juxtaposed in such a manner that their normal functions are maintained. The array is such that the promoter effects proper expression of the structural gene and the transcription termination signals effect proper termination of transcription and polyadenylation. The junction of these sequences may, for example, be effected by means of synthetic oligodeoxynucleotide linkers carrying the recognition sequence of an endonuclease.

The expression cassettes according to the invention may be inserted into the desired host in form of a stable plasmid or directly into the chromosome, of which the latter is preferred.

It is likewise possible that the expression plasmids include one or more, especially one or two, selective genetic markers for the host used for the expression.

As to the selective gene markers, any marker gene can be used which facilitates the selection for transformants due to the phenotypic expression of the marker gene.

Apart from the polypeptide expression cassette, and optionally replication origin(s) and genetic marker(s) the expression plasmids according to the invention can contain optionally additional expression cassettes, such as 1 to 3 additional polypeptide expression cassettes, which may be the same or different.

The expression plasmids are prepared by methods known in the art, for example by linking the polypeptide expression cassette, the DNA fragments containing selective genetic markers for the host used in the test and optionally for a bacterial host, the origin(s) of replication, and the optionally additional polypeptide expression cassettes in the predetermined order using conventional chemical or biological *in vitro* synthesis procedures. Preferentially, the plasmids are constructed and prepared using recombinant DNA techniques. For the preparation by recombinant DNA techniques suitable DNA fragments are ligated *in vitro* in conventional manner. The ligation mixture is then transformed into a suitable host depending on the nature of the regulatory elements used, and a transformant containing the desired vector is selected according to conventional procedures. The plasmids can be multiplicated by means of the transformed hosts and can be isolated in conventional manner. The choice of the host depends on the regulatory sequences located on the vector.

A suitable host for the production of the Ndr phosphokinase is preferably a eukaryotic cell, for example a mammalian, nematode, insect or yeast cell.

The suitable host, as defined above, can be transfected by the standard methods in genetic engineering, for example with the aid of a virus, lipid vesicles, particle gun or electroporation. To increase the amount of protein produced, it is advantageous to use a high copy plasmid or the plasmid DNA is integrated into the genome in several copies. The latter can be achieved, for example, through applying a selective stress, e.g., using methotrexate.

The transfected host cells can be cultured by standard methods in cell culture.

Once sufficient induction of Ndr phosphokinase is achieved, the protein can be purified by any of various methods known in the art (for example, see Scopes, 1987, Protein Purification: Principles and Practice, Second Edition, Springer-Verlag, N.Y.), if needed. During the isolation conventional additives like protein stabilisers, inhibitors of proteinases and the like may be added. For example, when the polypeptide is isolated from tissue culture, the first step consists usually in lysing the cells or, in the case where the polypeptide is secreted into the medium, in separating the cells from the culture fluid by means of centrifugation. In the presence of additional proteins and impurities, the resulting supernatant can be enriched for the polypeptide of the invention, e.g., by treatment with potyethyleneimine so as to remove most of the non-proteinaceous material, and precipitation of proteins by saturating the solution with ammonium sulphate or the like. Host proteins, if present, can also be precipitated by means of acidification with acetic acid and other conventional means. Other purification steps may include, for example, removing the lectins, desalination, chromatographic processes, such as ion exchange chromatography, gel filtration chromatography, partition chromatography, HPLC, reversed phase HPLC and the like. The separation of the constituents of the mixture is also effected by dialysis, according to charge by means of gel electrophoresis or carrier-free electrophoresis, according to molecular size by means of a suitable Sephadex column, gel-permeation or ultrafiltration, by affinity chromatography, or by other processes, especially those known from the literature.

Example 1 below describes a preferred embodiment, where the Ndr kinase is prepared as a fusion protein with an epitope-tag for ease of isolation. However, as is apparent to one of ordinary skill in the art in light of this disclosure, other appropriate purification methods can be used, also using native Ndr kinase.

The polypeptide, and especially its derivatives, may be obtained by synthetic means rather than derived from natural sources. Thus, using the information contained herein, Ndr polypeptide or peptides may be synthesised using commercially available protein synthesisers or even ordered from a commercial peptide synthesis service. Synthesised derivatives of Ndr may comprise any desired sequence modifications, including the use of altered amino acid residues or the addition of heterologous groups or side-chains to the polypeptide.

A combination of electrospray mass spectroscopy and metabolic cell labeling was used by the present inventors to map the phosphorylation sites of the isolated Ndr protein kinase. Two sites were identified as being phosphorylated, namely at a serine residue (Ser-281) and a threonine residue (Thr-444). The numbers assigned to these two residues reflect their position within the human Ndr kinase sequence as provided in WO 96/19579. Both of these sites, as well as the sequences surrounding them, are highly conserved in LATS, Orb6 and Cot-1, and are also present (albeit with slightly lower homology) in Dbf2, which are members of the Ndr subfamily of kinases, implicated in the regulation of cell division and cell morphology. Thus, the teachings of the present specification relating to Ndr kinase can be applied to other members of the Ndr subfamily of kinases.

In another aspect of the invention, functional homologues or fragments of Ndr phosphokinase are provided. As described above, these include derivatives and analogues of Ndr phosphokinase, including any single or multiple amino acid additions, substitutions and/or deletions occurring internally, or at the amino or carboxy termini thereof - so long as Ndr phosphokinase activity remains and at least Ser-281 or Thr-444, preferably both, are present. Particularly preferred homologues are those comprising at least one peptide defined by the sequence Arg-Xaa-Leu/Met-Ala-Xaa-P₁-Xaa-Val-GIy-Thr-Pro-Xaa-Tyr-Ile-Ala-Pro-Glu or Phe-Xaa-Xaa-Xaa-P₂-Xaa-Xaa-Xaa-Phe, where "/" indicates alternative amino acid residues, P₁ and P₂ refer to phosphorylated amino acid residues, preferably phosphoserine and phosphothreonine, respectively, Xaa can be any amino acid and the remaining amino acids are described by their conventional three letter codes. In a more preferred embodiment, the homologues comprise at least one peptide defined by the sequence Arg/Gln-Arg-Gln/Val/Leu-Leu/Met-Ala-Phe/Tyr/His-P₁-Thr/Leu-Val-Gly-Thr-Pro-Asp/Asn-Tyr-Ile-Ala-Pro-Glu or Phe-Ile/Leu/Phe-Glu/Gly/Asn-Phe/Tyr-P₂-Phe/Tyr-Lys/Arg-Lys/Arg-Phe, where where "/" indicates alternative amino acid residues, P₁ and P₂ refer to phosphorylated amino acid residues, preferably phosphoserine and phosphothreonine, respectively, and the remaining amino acids are described by their conventional three letter codes.

Also provided are peptides of at least 6 amino acids long comprising P₁ or P₂, and comprising consecutive amino acids selected from the formulae provided above. Preferred peptides are typically 6-15 amino acids long, depending on their projected use, with peptides of 8-10 amino acids long preferred for the production of antibodies, and peptides of 6 -10 or 6-15 amino acid long as phosphatase substrates, for example.

Thus, in a further aspect of the invention, antibodies specific for Ndr phosphokinase are provided. As described above, such antibodies are able to differentiate between the phosphorylated and non-phosphorylated forms of Ndr kinase. Such antibodies can be polyclonal, monoclonal, antibody fragments (Fc, Fv), and from any source, e.g., mouse, rat, goat, human, recombinant, etc. Such antibodies may be useful for identifying or isolating Ndr, for example by immunostaining or immunoseparation, or for disrupting Ndr activity *in vivo* or *in vitro*.

Ndr phosphokinase-specific antibodies may be prepared according to techniques known in the art. In order to prepare a polyclonal serum, for example, an antigenic portion of Ndr phosphokinase, consisting of a phosphopeptide derived therefrom or even the whole kinase, optionally in the presence of an adjuvant or conjugated to an immunostimulatory agent such as keyhole limpet haemocyanin, is injected into a mammal such as a mouse or a rabbit and antibodies are recovered therefrom by affinity purification using a solid-phase bound kinase or antigenic portion thereof. Monoclonal antibodies may be prepared according to similar established procedures.

Kinases such as Ndr are known to be involved in signal transduction within cells. This involvement makes kinases targets for agents which seek to obtain a biological effect by modulating a signalling pathway. Typically, modulation of a signalling pathway will alter the response of a cell to a particular stimulus. For example, the effect of hormones may be modulated by targeting the kinases involved in signal transduction from the hormone receptor to the biological effectors, which are typically regulators of gene expression.

The present inventors have demonstrated for the first time a functional requirement for phosphorylation in Ndr kinase activation. A number of strategies were followed in this respect, including the preparation of mutant Ndr kinase molecules having non-phosphorylatable residues and the use of specific phosphatases. For example, mutation of either the Ser-281 or Thr-444 sites to alanine strongly reduced both basal and okadaic acid-stimulated Ndr kinase activity, while combined mutation abolished Ndr kinase activity completely. See Example 2, below. In addition, protein phosphatase 2A was shown to dephosphorylate and inactivate Ndr, demonstrating that Ndr requires phosphorylation for its activity. See Example 3, below. Indeed, as Ndr kinase is potently activated when intact cells are treated with okadaic acid (a phosphatase 2A inhibitor), the protein phosphatase 2A may be a negative regulator of Ndr under physiological conditions. Thus, phosphorylation of Ser-281 and/or Thr-444 of Ndr protein kinase plays an important role in Ndr kinase activation.

Thus, in a further aspect of the invention, a method for identifying potential modulators of Ndr kinase activity is provided by contacting Ndr kinase or a functional homologue thereof with the potential modulator; and detecting a change in phosphorylation of the Ndr kinase or the functional homologue thereof. In an alternative embodiment, a method for identifying potential modulators of Ndr kinase activity is provided by contacting Ndr phosphokinase or a functional homologue thereof with the potential modulator; and detecting interaction between the modulator and Ndr phosphokinase or the functional homologue thereof. Such modulators can include without limitation agonists, antagonists and upstream members of a signaling pathway. In preferred embodiments, the Ndr phosphokinase or functional homologue thereof will be phosphorylated at Ser-281 or Thr-444 at greater than 0.1 mol/mol (phosphoserine/serine or phosphothreonine/threonine), more preferably greater than 0.2mol/mol (phosphoserine/serine or phosphothreonine/threonine), even more preferably greater than 0.3mol/mol (phosphoserine/serine or phosphothreonine/threonine). As demonstrated in the Examples below, the higher level of Ndr kinase phosphorylation provided by the methods of the present invention allow increased kinase activity.

Similarly, activated (i.e., phosphorylated) NDR kinase can be used to identify substrates. Thus, in a further aspect of the invention, a method of identifying an Ndr kinase substrate, is provided by incubating Ndr phosphokinase or a functional homologue thereof with the potential substrate; and detecting a change in phosphorylation of the Ndr kinase substrate. It has previously been shown that Ndr is capable of phosphorylating itself under artificial condition, however, the in vivo substrates of Ndr kinase are those which are encompassed by the present invention, providing a means to interfere with Ndr kinase activity.

Both the Ser-281 and Thr-444 sites (and also the surrounding sequences) are highly conserved in the kinase relatives of Ndr, suggesting a general mechanism of activation for kinases of the Ndr subfamily, distinct from PDK-1. NDR kinase appears to be involved in a novel pathway that may be of importance in oncology. Relatives of NDR are tumour suppressors. Given the materials and teachings of the present disclosure, one of ordinary skill in the art is therefore able to apply the knowledge to screen for modulators or substrates of other members of the Ndr kinase subfamily, as well as for Ndr kinase.

Thus, Ndr phosphokinase or its relatives, and their functional homologues can be useful as medicaments or for preparing medicaments, in particular for the treatment of abnormalities in cellular proliferation, such as cancer.

In addition, the materials of the present invention can be provided as kits for use in the screening methods of the invention, for example, or as substrates for phosphatases.

The following examples describe specific aspects of the invention to illustrate the invention and provide a description of the present method for those of skill in the art. The examples should not be construed as limiting the invention, as the examples merely provide specific methodology useful in understanding and practice of the invention.

Ndr phosphokinase materials can be used in the design of screening systems to identify putative therapeutic agents for use in treating anomalies in growth control. WO 97/18303, herein incorporated by reference, describes screening methodologies, kits, potential therapeutic agents and their formulation, the teachings of which can be applied to the materials and methods of the present invention.

### Examples

Standard methods in genetic engineering like random priming, subcloning, sequencing, cleavage with restriction enzymes, gel purification, ligations, transformation and annealing are carried out essentially as described in *Sambrook et al.*, Molecular Cloning: A laboratory manual, 2^{nd} Edn. Cold Spring Harbor Laboratory Press, Cold Spring Harbor NY, 1989.

### Example 1: Mapping of regulatory phosphorylation sites in Ndr

In this Example, cells were treated with the phosphatase inhibitor, okadaic acid, to induce higher levels of the Ndr phosphokinase to map the regulatory phosphorylation sites in Ndr.

Cell lines were maintained in Dulbecco's modified Eagle's medium (DMEM) containing 10% FCS, 100 U/ml penicillin and 100 µg/ml streptomycin unless otherwise indicated. African green monkey SV40 transformed kidney cells (COS-1 cells; available from the ATCC) were transfected with empty vector (pECE) or with a plasmid encoding HA-Ndr (pECE.HA.Ndr-WT) (Millward, T. A. et al. (1998) *EMBO J*. **17**, 5913-5922) using DEAE-dextran (Seed, B. and Aruffo, A. (1987) *Proc. Natl. Acad. Sci. U. S. A.* **84**, 3365-3369). COS-1 cells expressing HA-Ndr grown in serum-containing medium were treated for 1 h with 1 µM okadaic acid (Alexis Corporation) or with solvent alone (0.8% dimethylformamide; DMF). The monolayers were then washed once in ice-cold phosphate buffered saline (PBS), then lysed on the plate in IP buffer containing 1 µM microcystin (Millward, 1. A. et al. (1998)).

To immunopurify HA-Ndr for phosphorylation site analysis, extracts (10 - 15 mg) were mixed with the monoclonal antibody, 12CA5 (commercially available; Sells and Chemoff (1995) Gene 152, 187-9), prebound to protein A-Sepharose (∼200 µg 12CA5 bound to 100 µl beads). After washing, specifically bound proteins were eluted with 1 ml buffer containing 50 mM Tris-HCl pH 7.5, 0.1% NP-40, 4 µM leupeptin, 1 mM benzamidine, 1 µM microcystin and 1 mg/ml HA peptide (YPYDVPDYA). Eluted proteins were precipitated by the addition of 125 µg/ml sodium deoxycholate and 10% trichloroacetic acid (Bensadoun, A. and Weinstein, D. (1976) *Anal. Biochem.* **70**, 241-250), and redissolved in SDS-PAGE sample buffer. These samples were run out on a 7.5% preparative SDS gel, and bands corresponding to HA-Ndr excised. The protein bands can be visualized by staining with Coomassie blue.

Sodium dodecylsulfate- polyacrylamide gel electrophoresis (SDS-PAGE) of the immunopurified protein showed that HA-Ndr from okadaic acid-treated cells had a slightly slower electrophoretic mobility than did HA-Ndr from control cells, consistent with a higher level of phosphorylation. Approximately 1 - 2 µg of HA-Ndr obtained by this method was separated on a preparative gel, and the bands corresponding to HA-Ndr excised and digested in the gel slice with trypsin. The resultant mixture of peptides was analyzed by NanoESI mass spectrometry (MS) in a -79 precursor scan (Wilm, M. and Mann, M. (1996) Anal. Chem. 68, 1-8).This set-up is designed to measure the mass:charge ratios (m/z) of all peptides in the mixture that, upon fragmentation, liberate a species with a m/z of -79 (corresponding to a phosphate group).

In brief, gel slices containing HA-Ndr were reduced with dithiothreitol, alkylated with iodoacetamide and cleaved with trypsin (Promega, sequencing grade) as previously described (Shevchenko, A. et al. (1996) *Anal. Chem.* **68**, 850-858). NanoESI mass spectrometry (ESI-MS-MS) was performed according to a published method (Wilm, M. and Mann, M. (1996) *Anal. Chem.* **68,** 1-8) and phosphopeptides were detected by m/z 79 precursor ion scanning (Carr, S. A. et al. (1996) *Anal. Biochem.* **239,** 180-192). The mass spectra were acquired on an API 300 triple quadrupole mass spectrometer (PE Sciex, Toronto, Ontario, Canada) equipped with a NanoESI source (Protana, Odense, Denmark).

When HA-Ndr from control cells was analyzed in this way, several weak phosphopeptide signals could be detected. In HA-Ndr from okadaic acid-treated cells, however, the abundance of phosphopeptides was markedly increased. By referring to a theoretical digest of Ndr, the observed m/z of all of these peaks could be accounted for by five peptides, each of which was ∼79 Da (one phosphate group) heavier than would be expected for the non-phosphorylated state.

The five phosphopeptides identified as Ndr-derived peptides were namely:
QLAF**ST**VG**T**PDYIAPEVFMoxQTGYNK (P1; predicted/detected mass, Da 2794 /2873+79)
QLAF**ST**VG**T**PDYIAPEVFMoxQTGY (P2; predicted/detected mass, Da 2552 /2630+78)
QLAF**ST**VG**T**PDYIAPEVF (P3; predicted/detected mass, Da 1955 /2034+79)
DWVFIN**YTY**KR (P4; predicted/detected mass, Da 1505 /1585+80)
DWVFIN**YTY**K (P5; predicted/detected mass, Da 1349 /1429+80)

P1, P2 and P3 are derived from a region of the Ndr polypeptide encompassed by amino acid residues 277-301, whereas P4 and P5 are derived from a region of the Ndr polypeptide encompassed by amino acid residues 437-447. Potential phosphoacceptors in each peptide are marked in bold. The identities of several of these phosphopeptides were also confirmed by partial sequencing.

The first group of phosphopeptides (P1, P2 and P3) are from a region of the Ndr kinase domain that corresponds to the "activation-loop" (or "T-loop") of other kinases. The longest of these peptides is P1 (Ndr amino acids 277 - 301) and gave rise to three peaks in the -79 precursor scan (corresponding to the -3, -4 and -5 charged states). A methionine residue in this peptide was found to be oxidized; this oxidation presumably occurred during the purification procedure. Peptides P2 (amino acids 277 - 299) and P3 (amino acids 277 - 294) are shorter derivatives of P1, and appear to result from trace chymotryptic contamination of the trypsin preparation, since they both terminate with aromatic residues at the carboxy terminal end. As with P1, phosphopeptides P2 and P3 were also detected in multiple differentially charged states (-3, -4 and -5 for P2; -2, -3 and -4 for P3).

The second group of phosphopeptides comprises P4 and P5. Both of these derive from the carboxy terminal region of the Ndr polypeptide ∼60 amino acids C-terminal to the catalytic domain (amino acids 437 - 447), and are the result of alternative tryptic cleavages at the basic doublet Lys-446 / Arg-447. P4 gave rise to a single m/z peak (charge -2), while P5 was detected with charges of -2 and -3. Therefore, Ndr kinase from okadaic acid treated cells is phosphorylated on two distinct sites: one of these is located between amino acids 277 and 294, and the other one lies between amino acids 437 and 446.

With the microgram amounts of HA-Ndr used in these experiments, it was not possible to precisely define the phosphorylated residue within the two regions of Ndr that gave rise to phosphopeptides; note that each of these regions contain three amino acids that could potentially be phosphorylated (marked in bold). To locate the two phosphorylation sites precisely, HA-Ndr was isolated from cells that had been metabolically labeled with ³²P prior to okadaic acid treatment.

In brief, ten 10-cm plates of COS-1 cells were transfected with HA-Ndr as above. Seventy-two hours after transfection, the medium on the cells was changed to phosphate-free DMEM containing 10% dialyzed FCS and 1 mCi / plate of ³²Pᵢ (Amersham). The plates were incubated for a further 5 h and then stimulated for 1 h with 1 µM okadaic acid. After this, the cells were lysed and HA-Ndr immunopurified using 12CA5-Sepharose, electrophoresed on a preparative SDS gel, excised, and digested in the gel slice with trypsin as described above.

The ³²P-labelled tryptic peptides eluted from the gel slice were then separated by HPLC as described in Krieg et al. (1998). One phosphopeptide from each region (P1 from Ndr amino acids 277 - 294, and P4 from Ndr amino acids 437 - 446) was identified by on-line ES-MS and selected for further analysis. The fractions containing either phosphopeptide P1 or P4 were detected by LC-ESI-MS (Krieg et al., (1998)) and by their radioactivity. Each was analyzed for phosphoamino acid content, and was also subjected to Edman degradation, allowing determination of the cycle of ³²P release.

In brief, aliquots (200 c.p.m.) of each peak were lyophilized, resuspended in 6M HCl containing 0.2 mg/ml BSA, and incubated at 110°C for 45 min. Following this, the hydrolysate was lyophilized, redissolved in acetic acid/formic acid buffer (pH 1.9) together with unlabelled phosphoamino acid standards and separated by one-dimensional thin layer electrophoresis at pH 3.5 (Duclos, B. et al. (1991) *Methods Enzymol.* **201**, 10-21). After electrophoresis the plate was dried and exposed to a phosphorimager screen (Molecular Dynamics). In parallel, aliquots of the two purified ³²P-phosphopeptides (400 c.p.m.) were subjected to solid-phase Edman degradation using an automated model 477A sequenator (Applied Biosystems). Fractions from each cycle of Edman degradation were collected, lyophilized, redissolved in 20 µl of 50% acetonitrile, and then spotted onto a thin layer chromatography plate, before exposure to a phosphorimager screen. Ninhydrin-stained phosphoaminoacid standards can be used to identify the amino acids.

Purified phosphopeptide P1 gave rise exclusively to phosphoserine upon acid hydrolysis, and released radioactivity in the fifth cycle of Edman degradation, demonstrating phosphorylation of Ser-281. In contrast, phosphopeptide P4 contained phosphothreonine, and released ³²P in cycle 8 of Edman degradation, showing that Thr-444 is phosphorylated. These results demonstrate that cell stimulation with okadaic acid results in the phosphorylation of two sites in Ndr kinase, and that these two sites are Ser-281 and Thr-444.

In order to estimate the stoichiometry of phosphorylation of Ser-281 and Thr-444, a portion of the tryptic peptides were resolved by HPLC, and the HPLC profiles were scanned for both the phosphorylated and dephosphorylated forms of Ser-281- and Thr-444-containing peptides using online ES-MS (Krieg, J. et al. (1998) *Mol. Biol. Cell.* **9,** 301-309). In this way both phospho- and dephosphopeptides containing Ser-281 and Thr-444 could be detected as purified entities. The phosphopeptides were detected by extraction of the MS data for the corresponding ions as well as by Cerenkov counting of the LC fractions. Assuming equal elution efficiency of phospho- and dephospho forms, we were able to estimate that both Ser-281 and Thr-444 in Ndr from untreated cells (growing asynchronously in serum-containing medium) are phosphorylated to a stoichiometry of ∼0.1 mol/mol. This is consistent with the fact that HA-Ndr from unstimulated cells has a low but measurable level of basal activity. Upon okadaic acid treatment, Ser-281 phosphorylation rose to ∼0.8 mol/mol, while Thr-444 phosphorylation rose to ∼0.5 mol/mol.

Potential phosphorylation sites homologous to Ser-281 and Thr-444 of Ndr are present in several Ndr-related kinases. The catalytic domain homologies (amino acid identity) between Ndr and the kinases LATS, Orb6 and Cot-1 are 53%, 57% and 52%, respectively. The sequences surrounding Ser-281 and Thr-444 of Ndr and those corresponding to regions of LATS, Orb6 and Cot-1 also show homology:
- Ndr kinase:: RRQLAFSTVGTPDYIAPE
- Orb6:: RRVMAYSTVGTPDYIAPE
- LATS:: QRVLAHSLVGTPNYIAPE
- Cot-1: RRLMAYSTVGTPDYIAPE
and
- Ndr kinase:: FINYTYKRF
- Orb6:: FLGYTYKKF
- LATS:: FFEFTFRRF
- Cot-1: FIGYTFKRF.

### Example 2: Phosphorylation of Ser-281 and Thr-444 is required for Ndr activation.

To examine the functional requirement for Ser-281 and Thr-444 for Ndr kinase activity, mutants were created in which either one or both of these sites were replaced by alanine, to prevent phosphorylation.

In brief, the insert of the plasmid pECE.HA-Ndr.WT was cloned between the KpnI and XbaI sites of pcDNA3 (Invitrogen) to generate pcDNA3.HA-Ndr WT. To mutate Ser-281 or Thr-444 to either alanine, aspartic acid or glutamic acid, a 0.6 kb EcoRI - XbaI fragment from this plasmid (encoding Ndr amino acids 263 - 465) was subcloned into pBluescript and mutagenized using QuickChange (Stratagene) according to the manufacturer's instructions. The sequences of the mutated EcoRI - XbaI fragments were verified before cloning back into the parent plasmid (pcDNA3.HA-Ndr WT). Double mutants were generated by recombining the single mutants, using a unique StyI site located between the codons for Ser-281 and Thr-444 (nt 1650 of the Ndr cDNA).

COS-1 cells expressing wild-type HA-Ndr, HA-Ndr S281A, HA-Ndr T444A or HA-Ndr S281A/ T444A were treated for 1 h with solvent alone (DMF) or with 1 µM okadaic acid, as described in Example 1. HA-tagged kinases were then immunoprecipitated and assayed for kinase activity. Immune complex kinase assays for HA-Ndr and for endogenous Ndr were carried out as previously described (Millward, T. A. et al. (1998)).

Protein extracts (10 µg) were analyzed by immunoblotting with 12CA5 to verify similar expression levels of each Ndr construct. In brief, cell extracts were resolved by 7.5% SDS-PAGE and transferred to PVDF membranes (Immobilon, Millipore). Blots were blocked in Tris-buffered saline containing 1% Triton X-100, 0.5% Tween 20 and 5% non-fat milk powder (Fluka), and then probed with monoclonal antibodies 12CA5 or 9E10 (Immunoblotting - Cell extracts were resolved by 7.5% SDS-PAGE and transferred to PVDF membranes (Immobilon, Millipore). Blots were blocked in Tris-buffered saline containing 1% Triton X-100, 0.5% Tween 20 and 5% non-fat milk powder (Fluka), and then probed with monoclonal antibodies 12CA5 or 9E10 (commercially available; Sells and Chernoff (1995) Gene **152**,187-189), or with a polyclonal antibody against phosphorylated Ser-473 of PKB (New England Biolabs). Blots were developed with ECL using anti-rabbit IgG or anti-mouse IgG secondary antibodies from Amersham.

As in Example 1, HA-Ndr WT was potently (∼25-fold) stimulated by okadaic acid. Mutation of Ser-281 to alanine caused a marked reduction in basal kinase activity. The S281A mutant, however, was still activated to some extent by okadaic acid. Mutation of Thr-444 to alanine caused similar effects: both basal and okadaic acid-stimulated activity were reduced, but activity was still stimulated by okadaic acid. However, combined mutation of the two sites (S281A/T444A) reduced basal activity to near undetectable levels, and abolished the ability of Ndr kinase to be stimulated by okadaic acid. Western blot analysis confirmed that the various mutants were expressed at comparable levels. These data indicate a) that both Ser-281 and Thr-444 phosphorylation is needed for full Ndr activity, b) that phosphorylation of either site alone is sufficient to support a more limited but nevertheless measurable degree of Ndr activity, and c) that neither site depends on the other for phosphorylation.

We also attempted to create constitutively active mutants of Ndr by replacing Ser-281 and/or Thr-444 with negatively charged amino acids. Replacement of Ser-281 with either aspartic acid or glutamic acid resulted in basal and okadaic acid-stimulated activities similar to those of the S281A mutant (see above), suggesting that steric constraints in this part of the molecule are too tight to allow effective mimicry of a phosphate group by a negatively charged amino acid side chain (data not shown). Replacement of Thr-444 with either Asp or Glu, on the other hand, yielded mutants with moderately (1.5 to 2-fold) elevated basal kinase activity.

As described in Example 1, Ser-281 and Thr-444 are conserved in Ndr-related kinases including the kinases LATS, Orb6, Cot-1 and Dbf2. Alignment of the sequences of these kinases shows that potential phosphorylation sites corresponding to both Ser-281 and Thr-444 are conserved throughout the family. Moreover, the sequences surrounding these sites are highly conserved (in particular, LATS, Orb-6 and Cot-1). It is therefore probable that the kinase activity of the Ndr related kinases are also regulated by phosphorylation on sites equivalent to Ser-281 and Thr-444 of Ndr. Ndr protein kinase is the closest known relative of the tumor suppressor LATS in mammalian cells. Ndr and LATS are coexpressed in several tissues and cell types, and their high level of homology in regions targeted for phosphorylation in Ndr suggests the possibility that they could be regulated by the same upstream kinase.

### Example 3: Negative regulation of Ndr protein kinase by a type 2A-phosphatase in vivo and in vitro.

COS-1 cells were transfected with a plasmid encoding HA-Ndr as described above, and were then treated for one hour with okadaic acid (1 µM; Alexis Corporation), calyculin A (10 nM; Alexis Corporation) or cyclosporin A (1 µM; Biomol), or solvent alone (DMF). Stock solutions of the phosphatase inhibitors were dissolved in dimethyl formamide, and were added to cells in serum-containing medium. The behavior of these compounds in intact cells has been studied extensively in previous work. When applied to cells at the concentrations stated above, okadaic acid effectively inhibits intracellular type 2A phosphatases, without affecting protein phosphatase 1 (Favre, B. et al. (1997) *J. Biol. Chem.* **272**, 13856-13863); calyculin A is about half as potent as okadaic acid in inhibiting PP2A, and also inhibits protein phosphatase 1 activity, while cyclosporin A acts specifically on calcineurin (Liu, J. et al. (1991) *Cell* **66**, 807-815). Following the treatment, the activity of HA-Ndr was measured in an immune complex kinase assay, as described in Example 2.

Okadaic acid treatment of COS-1 caused a >10-fold activation of HA-Ndr. Calyculin A, on the other hand, was only half as potent as okadaic acid in activating Ndr. Treatment with cyclosporin A had no effect on Ndr activity, although previous studies have shown that COS-1 cells express calcineurin, and respond to cyclosporin A treatment with hyperphosphorylation of Elk-1 (Sugimoto, T. et al. (1997) *J. Biol. Chem.* **272**, 29415-29418). The profile of activity of these compounds in stimulating Ndr suggests that a type 2A-phosphatase (most likely PP2A itself) acts as a negative regulator of Ndr, whereas protein phosphatase 1 and calcineurin are unlikely to participate in the regulation of Ndr activity.

The observation that inhibition of PP2A is sufficient to cause activation of Ndr in intact cells suggests that PP2A might act directly as a negative regulator of Ndr. However it is also possible that okadaic acid causes Ndr activation through an indirect mechanism. To address this issue, HA-Ndr was immunoprecipitated from either control cells or okadaic acid-stimulated cells, and was then incubated in vitro with purified PP2A core dimer (which consists of the 36 kDa catalytic subunit complexed with the PR65 regulatory subunit).

In brief, HA-Ndr was immunoprecipitated and then washed in phosphatase IP buffer (50 mM Tris-HCl pH 7.5, 1% *β*-mercaptoethanol, 1 mM MnCl₂, 0.5 mM phenylmethylsulfonyl fluoride and 1 mM benzamidine). The immunoprecipitates were divided into equal portions and incubated for 1 h at 30°C with phosphatase buffer alone, phosphatase buffer containing PP2A core dimer (0.1 U, where 1 U releases 1 nmol Pᵢ / min from phosphorylase a), or phosphatase buffer containing PP2A core dimer plus 100 nM okadaic acid. The core dimer of protein phosphatase 2A (containing 36 kDa and 65 kDa subunits) can be purified from rabbit skeletal muscle (Hemmings et al., (1990) Biochemistry Apr 3;29(13):3166-73). After incubation with the buffer/phosphatase/phosphatase and okadaic acid, the beads were washed with IP buffer, then with IP buffer containing 1 M NaCl, and finally with 20 mM Tris-HCl pH 7.5 containing 4 µM leupeptin and 1 mM benzamidine. Then, the kinase activity of Ndr was determined in the standard peptide kinase assay.

Regardless of whether HA-Ndr had been isolated from untreated or from okadaic acid-stimulated cells, incubation together with PP2A in vitro completely abolished its kinase activity. This inactivation of Ndr depended upon the phosphatase activity of PP2A (and not, for example, upon contaminating proteolytic activity) because inclusion of okadaic acid in the incubation mixture prevented Ndr inactivation. PP2A also inactivated HA-Ndr isolated from A23187-treated cells, confirming earlier data suggesting that activation of Ndr by a calcium ionophore is a consequence of S100 protein-stimulated autophosphorylation. Taken together, these results demonstrate that Ndr is completely dependent upon phosphorylation for its kinase activity, and also suggest that PP2A might be a physiological regulator of Ndr in vivo.

### Example 4: Evidence that Ndr is not a target of PDK-1.

Ser-281 of Ndr shows striking homology to the activation-loop phosphorylation sites that are found in several members of the AGC subgroup of serine/threonine kinases, such as PKB/Akt (Alessi, D. R., et al. (1997) *Curr. Biol.* **7**, 776-789), p70 S6 kinase (Pullen et al., (1998) Science **279**, 707-710; Alessi, D. R. et al. (1998) *Curr. Biol.* **8**, 69-81), SGK (Kobayashi, T. and Cohen, P. (1999) *Biochem. J.* **339**, 319-328; Park, J. et al. (1999) *EMBO J.* **18**, 3024-3033), protein kinase C (Dutil, E. M. et al. (1998) *Curr. Biol.* **8**, 1366-1375; Le Good, J. A. et al. (1998) *Science* **281**, 2042-2045 ) and cAMP-dependent protein kinase (Cheng, X. et al. (1998) *Proc. Natl. Acad. Sci. U. S. A.* **95**, 9849-9854). The site equivalent to Ser-281 in each of these kinases is a substrate for the phosphoinositide-dependent kinase PDK-1. Based on sequence comparisons of PKB/Akt, p70 S6 kinase, protein kinase C and cAMP-dependent protein kinase, Cheng and co-workers proposed a consensus phosphorylation motif for PDK-1 as Thr-Xaa-Cys-Gly-Thr-Xaa-Asp/Glu-Tyr-Xaa-Ala-Pro-Glu, where Xaa is a hydrophobic residue, and the first Thr in the sequence is the phosphoacceptor (Cheng, X. et al., (1998)). Strikingly, the sequence around Ser-281 of Ndr is homologous to this consensus in nine out of twelve positions.

Moreover, most PDK-1 targets contain a second phosphorylation site approximately 60 amino acids carboxy-terminal to the end of the kinase catalytic domain, which is flanked by hydrophobic amino acids (consensus Phe-Xaa-Xaa-Phe-Ser/Thr-Phe/Tyr) and which, at least in the case of PKB, is phosphorylated by a modified form of PDK-1 (Balendran, A et al. (1999) *Curr. Biol.* **9**, 393-404). Thr-444 of Ndr shows similarity to this second motif both in terms of the sequence surrounding it and in terms of its positioning within the polypeptide chain. We therefore considered the possibility that Ndr could also be a target of PDK-1.

To evaluate this possibility, myc-tagged PDK-1 was coexpressed with HA-Ndr in human embryonic kidney cells (HEK-293 cells transformed with fragments of adenovirus type 5 DNA; available from the ATCC), and Ndr was then immunoprecipitated and assayed for kinase activity. For comparison, myc-PDK-1 was cotransfected with PKB, an established PDK-1 substrate. HEK-293 cells were transfected with pCMV5.myc-PDK-1 (0, 1 or 5 µg of plasmid; Pullen, N. et al. (1998) *Science* 279, 707-710) and pECE.HA.Ndr-WT (5 µg DNA) or pECE.HA-ΔPH-PKB (5 µg DNA) (Andjelkovic, M. et al. (1999) *Mol. Cell. Biol.* 19, 5061-5072) using a modified calcium phosphate procedure (Chen, C. and Okayama, H. (1987) *Mol. Cell. Biol.* **7**, 2745-2752). After transfection, cells were serum-starved for 24h, and then HA-tagged kinases were immunoprecipitated and assayed for kinase activity as described above, except that 50 µg (0.1 mg/ml) cell extract was used, and the kinase substrate used was Crosstide (GRPRTSSFAEG; 30 µM). Protein extracts (10 µg) were immunoblotted with 12CA5 to verify expression of HA-Ndr and HA-ΔPH-PKB, or with 9E10 to verify expression of myc-PDK. Immunoblotting was carried out as described above except a polyclonal antibody against phosphorylated Ser-473 of PKB (New England Biolabs) was used.

HEK-293 cells were grown for 24h in medium lacking serum, and were then left untreated (Control), or were treated with 100 nM insulin or 50 ng/ml IGF-1 for 10 min. Alternatively, asynchronously growing HEK-293 cells were treated with 0.8% DMF (Control) or with 1 µM okadaic acid for 1 h. Endogenous Ndr activity was then determined in each cell extract by immune complex kinase assay. The cell extracts (30 µg per lane) were immunoblotted with an antibody that recognizes the activated form of PKB, phosphorylated on Ser-473.

PDK-1 expression had very little effect on HA-Ndr activity although it was readily detectable by western blotting. In contrast, PDK-1 expression caused a >20-fold activation of ΔPH-PKB. Thus, Ndr does not appear to be activated by overexpression of PDK-1.

Some PDK-1 substrates require prior modification by another molecule before they can be optimally phosphorylated by PDK-1. PKB, for example, is only an efficient PDK-1 substrate when phosphoinositides are bound to its PH domain, whereas p70 S6 kinase must first be phosphorylated on a distal site. One form of Ndr regulation that we have characterized previously involves the Ca²⁺-dependent binding of S100 proteins to the N-terminal regulatory domain of Ndr (Millward et al., (1998)). To investigate whether PDK-1 might require such a modification of Ndr in order to phosphorylate it, myc-PDK-1 was immunoprecipitated and incubated in a kinase reaction with [γ³²P]-ATP and a complex of GST-Ndr K118A and Ca²⁺/S100B. However, myc-PDK-1 failed to phosphorylate the complex of GST-Ndr K118A with S100B in vitro, although efficient autophosphorylation of PDK-1 was observed.

Several PDK-1 substrates, including PKB, SGK and p70 S6 kinase, are potently activated when cells are exposed to insulin or IGF-1. We investigated whether these ligands are able to activate Ndr in HEK-293 cells, Insulin and IGF-1 are commercially available (e.g., from Boehringer Mannheim) and cells were serum-starved in DMEM lacking serum for 24 h prior to stimulation with these agonists.

Endogenous Ndr was activated ∼15-fold by OA, but showed no response to either insulin or IGF-1. The same extracts used to measure Ndr kinase activity were immunoblotted using a phosphospecific antibody that recognizes phosphorylated Ser-473 of PKB (which is similar to Thr-444 of Ndr). As expected, phosphorylation of PKB Ser-473 was strongly induced by both insulin and IGF-1. The lack of activation of Ndr by insulin or IGF-1 was confirmed using epitope-tagged Ndr overexpressed in HEK-293 cells. Since Ndr was not activated under conditions that promote phosphorylation and activation of PKB, it would appear that the kinase that activates Ndr is distinct from the kinase responsible for phosphorylation of PKB.

Thus, surprisingly, Ndr is probably not a target of the phosphoinositide-dependent protein kinase PDK-1, although the regulatory phosphorylation sites in Ndr show extensive similarity to those of several other kinases known to be regulated by PDK-1. Although not wishing to be bound by theory, one potential explanation for the lack of recognition of Ndr by PDK-1 could be an unusual structural feature present in Ndr. Ndr contains an insert of ∼40 amino acids between the catalytic subdomains VII and VIII, which is not present in any other known kinases except for those closely related to Ndr. This insert interrupts the canonical kinase catalytic domain, beginning C-terminally to the conserved "DFG" kinase motif, and ending directly N-terminal to Ser-281. In Ndr, this insert appears to harbor a nuclear localization signal (Millward et al., (1995)). Conceivably, the close proximity of this domain to Ser-281 could influence the recognition of Ser-281 by upstream kinases, such that phosphorylation by PDK-1 is prevented, or this domain might actively recruit an upstream kinase to the Ndr activation loop.

## Claims

1. Nuclear, Dbf2-related (Ndr) phosphokinase, a functional homologue thereof or a modulator thereof, wherein said Ndr phosphokinase is phosphorylated at Ser-281 and/or Thr-444.

2. Ndr phosphokinase of claim 1, wherein said phosphorylation at Ser-281 or Thr-444 is greater than 0.1 mol/mol (phosphoserine/serine or phosphothreonine/threonine).

3. Ndr phosphokinase of claim 1, wherein said phosphorylation at Ser-281 or Thr-444 is greater than 0.2mol/mol (phosphoserine/serine or phosphothreonine/threonine)

4. The functional homologue of claim 1, wherein said homologue comprises at least one peptide defined by the sequence:
Xaa-Arg-Xaa-Leu/Met-Ala-Xaa-P₁-Xaa-Val-Gly-Thr-Pro-Xaa-Tyr-Ile-AIa-Pro-Glu
or
Phe-Xaa-Xaa-Xaa-P₂-Xaa-Xaa-Xaa-Phe.

5. An antibody that specifically binds to Ndr phosphokinase or a functional homologue thereof.

6. The antibody of claim 5, wherein said antibody is monoclonal.

7. A method for producing the Ndr phosphokinase of claim 1, 2 or 3, said method comprising treating cells containing a nucleic acid encoding Ndr protein kinase with okadaic acid, and allowing said cells to express said Ndr protein kinase.

8. The method according to claim 7, said method further comprising the step of purifying said kinase.

9. The use of Ndr phosphokinase of claim 1, 2 or 3, or a functional homologue thereof, for the identification of an agonist or antagonist of Ndr kinase activity.

10. The use of Ndr phosphokinase of claim 1, 2 or 3, a functional homologue thereof or a modulator therof, as a medicament.

11. The use of Ndr phosphokinase of claim 1, 2 or 3, a functional homologue thereof or a modulator therof, in the treatment of abnormalities in cellular proliferation.

12. A method for screening potential modulators of Ndr kinase activity, said method comprising the steps of:
a) incubating Ndr phosphokinase or a functional homologue thereof with the potential modulator; and
b) detecting interaction between the modulator and Ndr phosphokinase or the functional homologue thereof.

13. A method for screening potential modulators of Ndr kinase activity, said method comprising the steps of
a) incubating Ndr kinase or a functional homologue thereof with the potential modulator; and
b) detecting a change in phosphorylation of said Ndr kinase or the functional homologue thereof.

14. The process according to claim 13, wherein said potential modulator is a kinase of a signalling pathway.

15. A kit comprising:
Ndr phosphokinase or a functional fragment thereof; and instructions.

16. A method of identifying an Ndr kinase substrate, said method comprising the steps of:
(a) incubating Ndr phosphokinase or a functional homologue thereof with the potential substrate; and
(b) detecting a change in phosphorylation of said Ndr kinase substrate, with the proviso that said Ndr kinase substrate is not Ndr kinase.

17. A phosphokinase of the subfamily of Ndr-related phosphokinases, a functional homologue thereof or a modulator thereof, wherein said phosphokinase is phosphorylated at amino acids Ser-281 and/or Thr-444 of Ndr protein kinase or amino acids corresponding to amino acids Ser-281 and/or Thr-444 of Ndr protein kinase.


### Annex to the application documents ― subsequently filed sequence listing

SEQUENCE LISTING

<110> Novartis AG

<120> Organic Compounds

<130> 4-31137/A

<140> GB 9921121.1
<141> 1999-09-07

<160> 20

<170> PatentIn version 3.0

<210> 1
<211> 17
<212> PRT
<213> unidentified

<220>
<221> MOD_RES
<222> (3)..(3)
<223> Leu or Met


<220>
<221> MOD_RES
<222> (6)..(6)
<223> PHOSPHORYLATION- especially phosphoserine


<400> 1

Arg Xaa Xaa Ala Xaa Xaa Xaa Val Gly Thr Pro Xaa Tyr Ile Ala Pro
1               5                   10                  15

Glu


<210> 2
<211> 18
<212> PRT
<213> unidentified

<220>
<221> MOD_RES
<222> (4)..(4)
<223> Leu or Met


<220>
<221> MOD_RES
<222> (7)..(7)
<223> PHOSPHORYLATION-especially phosphoserine

<400>  2

Xaa Arg Xaa Xaa Ala Xaa Xaa Xaa Val Gly Thr Pro Xaa Tyr Ile Ala
1               5                   10                  15

Pro Glu

<210>  3
<211>  9
<212>  PRT
<213>  unidentified

<220>
<221>  MOD_RES
<222>  (5)..(5)
<223>  PHOSPHORYLATION-especially phosphothreonine

<400>  3

Phe Xaa Xaa Xaa Xaa Xaa Xaa Xaa Phe
1               5

<210>  4
<211>  18
<212>  PRT
<213>  unidentified

<220>
<221>  MOD_RES
<222>  (1)..(1)
<223>  Arg or Gln

<220>
<221>  MOD_RES
<222>  (3)..(3)
<223>  Gln or Val or Leu

<220>
<221>  MOD_RES
<222>  (4)..(4)
<223>  Leu or Met

<220>
<221>  MOD_RES
<222>  (6)..(6)
<223>  Phe or Tyr or His

<220>
<221>  MOD_RES

<222>  (7)..(7)
<223>  PHOSPHORYLATION-especially phosphoserine


<220>
<221>  MOD_RES
<222>  (8)..(8)
<223>  Thr or Leu


<220>
<221>  MOD_RES
<222>  (13)..(13)
<223>  Asp or Asn


<400>  4

Xaa Arg Xaa Xaa Ala Xaa Xaa Xaa Val Gly Thr Pro Xaa Tyr Ile Ala
1               5                   10                  15

Pro Glu


<210>  5
<211>  9
<212>  PRT
<213>  unidentified

<220>
<221>  MOD_RES
<222>  (2)..(2)
<223>  Ile or Leu or Phe


<220>
<221>  MOD_RES
<222>  (3)..(3)
<223>  Glu or Gly or Asn


<220>
<221>  MOD_RES
<222>  (4)..(4)
<223>  Phe or Tyr


<220>
<221>  MOD_RES
<222>  (5)..(5)
<223>  PHOSPHORYLATION-especially phosphothreonine


<220>
<221>  MOD_RES
<222>  (6)..(6)

<223>  Phe or Tyr

<220>
<221>  MOD_RES
<222>  (7)..(7)
<223>  Lys or Arg

<220>
<221>  MOD_RES
<222>  (8)..(8)
<223>  Lys or Arg

<400>  5

Phe Xaa Xaa Xaa Xaa Xaa Xaa Xaa Phe
1               5

<210>  6
<211>  25
<212>  PRT
<213>  unidentified

<220>
<221>  MOD_RES
<222>  (5)..(5)
<223>  phosphoacceptor

<220>
<221>  MOD_RES
<222>  (6)..(6)
<223>  phosphoacceptor

<220>
<221>  MOD_RES
<222>  (9)..(9)
<223>  phosphoacceptor

<220>
<221>  MOD_RES
<222>  (19)..(19)
<223>  Mox- phosphoacceptor

<400>  6

Gln Leu Ala Phe Ser Thr Val Gly Thr Pro Asp Tyr Ile Ala Pro Glu
1               5                   10                  15

Val Phe Met Gln Thr Gly Tyr Asn Lys
            20                  25

<210>  7
<211>  23
<212>  PRT
<213>  unidentified

<220>
<221>  MOD_RES
<222>  (5)..(5)
<223>  phosphoacceptor


<220>
<221>  MOD_RES
<222>  (6)..(6)
<223>  phosphoacceptor


<220>
<221>  MOD_RES
<222>  (9)..(9)
<223>  phosphoacceptor


<220>
<221>  MOD_RES
<222>  (19)..(19)
<223>  phosphoacceptor


<400>  7

Gln Leu Ala Phe Ser Thr Val Gly Thr Pro Asp Tyr Ile Ala Pro Glu
1               5                   10                  15

Val Phe Met Gln Thr Gly Tyr
            20

<210>  8
<211>  18
<212>  PRT
<213>  unidentified

<220>
<221>  MOD_RES
<222>  (5)..(5)
<223>  phosphoacceptor


<220>
<221>  MOD_RES
<222>  (6)..(6)
<223>  phosphoacceptor


<220>

<221>  MOD_RES
<222>  (9)..(9)
<223>  phosphoacceptor

<400>  8

Gln Leu Ala Phe Ser Thr Val Gly Thr Pro Asp Tyr Ile Ala Pro Glu
1               5                   10                  15

Val Phe

<210>  9
<211>  11
<212>  PRT
<213>  unidentified

<220>
<221>  MOD_RES
<222>  (7)..(9)
<223>  phosphoacceptor

<400>  9

Asp Trp Val Phe Ile Asn Tyr Thr Tyr Lys Arg
1               5                   10

<210>  10
<211>  10
<212>  PRT
<213>  unidentified

<400>  10

Asp Trp Val Phe Ile Asn Tyr Thr Tyr Lys
1               5                   10

<210>  11
<211>  18
<212>  PRT
<213>  unidentified

<220>
<221>  misc_feature
<223>  catalytic domain of Ndr kinase

<400>  11

Arg Arg Gln Leu Ala Phe Ser Thr Val Gly Thr Pro Asp Tyr Ile Ala
1               5                   10                  15

Pro Glu

<210>  12
<211>  18
<212>  PRT
<213>  unidentified

<220>
<221>  misc_feature
<223>  catalytic domain of Orb 6


<400>  12

Arg Arg Val Met Ala Tyr Ser Thr Val Gly Thr Pro Asp Tyr Ile Ala
1               5                   10                  15

Pro Glu


<210>  13
<211>  18
<212>  PRT
<213>  unidentified

<220>
<221>  misc_feature
<223>  catalytic domain of Lats


<400>  13

Gln Arg Val Leu Ala His Ser Leu Val Gly Thr Pro Asn Tyr Ile Ala
1               5                   10                  15

Pro Glu


<210>  14
<211>  18
<212>  PRT
<213>  unidentified

<220>
<221>  misc_feature
<223>  catalytic domain of Cot-1


<400>  14

Arg Arg Leu Met Ala Tyr Ser Thr Val Gly Thr Pro Asp Tyr Ile Ala
1               5                   10                  15

Pro Glu


<210>  15

<211>  9
<212>  PRT
<213>  unidentified

<220>
<221>  misc_feature
<223>  catalytic domain of Ndr kinase


<400>  15

Phe Ile Asn Tyr Thr Tyr Lys Arg Phe
1               5

<210>  16
<211>  9
<212>  PRT
<213>  unidentified

<220>
<221>  misc_feature
<223>  catalytic domain of Orb 6


<400>  16

Phe Leu Gly Tyr Thr Tyr Lys Lys Phe
1               5

<210>  17
<211>  9
<212>  PRT
<213>  unidentified

<220>
<221>  misc_feature
<223>  catalytic domain of Lats


<400>  17

Phe Phe Glu Phe Thr Phe Arg Arg Phe
1               5

<210>  18
<211>  9
<212>  PRT
<213>  unidentified

<220>
<221>  misc_feature
<223>  catalytic domain of Cot-1


<400>  18

Phe Ile Gly Tyr Thr Phe Lys Arg Phe
1               5

<210>  19
<211>  12
<212>  PRT
<213>  unidentified

<220>
<221>  MOD_RES
<222>  (1)..(1)
<223>  phosphoacceptor


<220>
<221>  misc_feature
<223>  Xaa is a hydrophobic residue


<400>  19

Thr Xaa Cys Gly Thr Xaa Xaa Tyr Xaa Ala Pro Glu
1               5                   10

<210>  20
<211>  6
<212>  PRT
<213>  unidentified

<220>
<221>  misc_feature
<223>  X = unidentifed amino acid


<400>  20

Phe Xaa Xaa Phe Xaa Xaa
1               5